# EUROPÄISCHE PATENTANMELDUNG

(11) **EP 4 064 578 A1**
(43) Veröffentlichungstag der Anmeldung: **28.09.2022**
(21) Anmeldenummer: 21165308.4
(22) Anmeldetag: 26.03.2021
(51) Int. Cl.: H04B 5/00, H04B 13/00, H01L 35/28, H04W 12/00, A61B 5/00

(54) **BAND UND BETRIEBSVERFAHREN FÜR EIN BAND**

(71) Anmelder: BKS GmbH, 42549 Velbert (DE)
(72) Erfinder: Braam, Reinhold, 46414 Rhede (DE); Bühe, Jonas, 42549 Velbert (DE)
(74) Vertreter: DREISS Patentanwälte PartG mbB

(57) **Zusammenfassung**

Band zur Befestigung an einem Körper, beispielsweise an einer Extremität, beispielsweise eines Säugetiers und/oder einer Person, beispielsweise Armband, aufweisend eine Kommunikationseinrichtung, die dazu ausgebildet ist, eine kapazitive Datenübertragung, beispielsweise mit wenigstens einer weiteren Einrichtung, auszuführen.

## Beschreibung

Die Offenbarung bezieht sich auf ein Band zur Befestigung an einem Körper.

Die Offenbarung bezieht sich ferner auf ein Verfahren zum Betreiben eines Bands zur Befestigung an einem Körper.

Beispielhafte Ausführungsformen beziehen sich auf ein Band zur Befestigung an einem Körper, beispielsweise an einer Extremität, beispielsweise eines Säugetiers und/oder einer Person, beispielsweise Armband, aufweisend eine Kommunikationseinrichtung, die dazu ausgebildet ist, eine kapazitive Datenübertragung, beispielsweise mit wenigstens einer weiteren Einrichtung, auszuführen.

Weitere beispielhafte Ausführungsformen stellen somit eine am Körper tragbare bzw. mobile Einrichtung zur Ausführung einer kapazitiven Datenübertragung bereit.

Bei weiteren beispielhaften Ausführungsformen ist das Band als Armband ausgebildet, also beispielsweise am bzw. um einen Arm angeordnet tragbar.

Bei weiteren beispielhaften Ausführungsformen ist das Band als Fußband ausgebildet, also beispielsweise am bzw. um einen Fuß bzw. ein Bein angeordnet tragbar.

Bei weiteren beispielhaften Ausführungsformen ist das Band als Halsband ausgebildet, also beispielsweise am bzw. um den Hals angeordnet tragbar.

Bei weiteren beispielhaften Ausführungsformen kann das Band z.B. von einer Person getragen werden.

Bei weiteren beispielhaften Ausführungsformen kann das Band z.B. von einem Tier, beispielsweise einem Säugetier, getragen werden.

Bei weiteren beispielhaften Ausführungsformen ist vorgesehen, dass die Kommunikationseinrichtung einen Sender oder einen Transceiver (Sendeempfänger) für die kapazitive Datenübertragung aufweist.

Bei weiteren beispielhaften Ausführungsformen ist vorgesehen, dass das Band wenigstens eine Antenne für die kapazitive Datenübertragung aufweist.

Bei weiteren beispielhaften Ausführungsformen ist vorgesehen, dass das Band eine Energieversorgungseinrichtung zur zumindest zeitweisen Versorgung wenigstens einer Komponente des Bands mit elektrischer Energie aufweist.

Bei weiteren beispielhaften Ausführungsformen ist vorgesehen, dass die Energieversorgungseinrichtung einen Energiespeicher, beispielsweise zur Speicherung elektrischer Energie, aufweist, wobei beispielsweise der Energiespeicher wenigstens einen Superkondensator aufweist oder als Superkondensator ausgebildet ist. Alternativ oder ergänzend zu dem wenigstens einen Superkondensator kann bei weiteren beispielhaften Ausführungsformen auch wenigstens eine Batterie bzw. ein Akkumulator vorgesehen sein.

Bei weiteren beispielhaften Ausführungsformen ist vorgesehen, dass das Band einen thermoelektrischen Energiewandler aufweist, der beispielsweise gemäß dem Seebeck-Effekt arbeitet, und/oder der beispielsweise dazu ausgebildet ist, einen bzw. den Energiespeicher aufzuladen. Auf diese Weise kann z.B. die Körperwärme eines Trägers des Bandes genutzt werden, um elektrische Energie für den Betrieb der wenigstens einen Komponente des Bands und/oder für das Aufladen des Energiespeichers bereitzustellen.

Bei weiteren beispielhaften Ausführungsformen ist vorgesehen, dass das Band einen Schichtaufbau aufweist, wobei der Schichtaufbau aufweist: eine erste Schicht mit einer ersten Wärmeleitfähigkeit, eine zweite Schicht mit einer zweiten Wärmeleitfähigkeit, und eine dritte Schicht mit einer dritten Wärmeleitfähigkeit, wobei die dritte Schicht zwischen der ersten Schicht und der zweiten Schicht angeordnet ist, wobei beispielsweise die erste Schicht und die zweite Schicht mit dem Energiewandler, beispielsweise thermisch leitfähig, verbindbar bzw. verbunden ist. Dies ermöglicht bei weiteren beispielhaften Ausführungsformen vorteilhaft eine effiziente Bereitstellung eines Temperaturunterschieds für den thermoelektrischen Energiewandler.

Bei weiteren beispielhaften Ausführungsformen ist vorgesehen, dass die erste Wärmeleitfähigkeit und/oder die zweite Wärmeleitfähigkeit größer ist als die dritte Wärmeleitfähigkeit, beispielsweise wenigstens um einen Faktor zehn, beispielsweise wenigstens um einen Faktor 100.

Bei weiteren beispielhaften Ausführungsformen ist vorgesehen, dass die erste Schicht und die zweite Schicht aus einem thermisch gut leitenden Material ausgebildet sind bzw. ein thermisch gut leitendes Material aufweisen. Bei weiteren beispielhaften Ausführungsformen können die erste und zweite Schicht auch unterschiedliche Materialien aufweisen.

Bei weiteren beispielhaften Ausführungsformen ist vorgesehen, dass die dritte Schicht ein thermisch nicht gut leitendes, also z.B. isolierendes, Material aufweist. Bei weiteren beispielhaften Ausführungsformen ist vorgesehen, dass die dritte Schicht dazu ausgebildet ist die erste Schicht thermisch von der zweiten Schicht zu isolieren.

Bei weiteren beispielhaften Ausführungsformen ist vorgesehen, dass die erste Schicht dazu ausgebildet ist, Wärmeenergie von einem Körper eines Trägers des Bandes zu dem Energiewandler, beispielsweise zu einem ersten Anschluss des Energiewandlers hin, zu leiten, beispielsweise um den ersten Anschluss des Energiewandlers zu erwärmen.

Bei weiteren beispielhaften Ausführungsformen ist vorgesehen, dass die zweite Schicht dazu ausgebildet ist, Wärmeenergie von dem Energiewandler, beispielsweise von einem zweiten Anschluss des Energiewandlers, weg zu leiten, beispielsweise zu einer Umgebung, beispielsweise um den zweiten Anschluss des Energiewandlers zu kühlen.

Bei weiteren beispielhaften Ausführungsformen ist vorgesehen, dass das Band wenigstens eine Steuereinrichtung zur zumindest zeitweisen Steuerung eines Betriebs wenigstens einer Komponente aufweist. Bei weiteren beispielhaften Ausführungsformen ist vorgesehen, dass die Steuereinrichtung einen Mikrocontroller oder einen programmierbaren Logikbaustein oder dergleichen aufweist. Bei weiteren beispielhaften Ausführungsformen ist vorgesehen, dass das Band ein Gehäuse aufweist, wobei wenigstens eine der Komponenten des Bands in und/oder an dem Gehäuse angeordnet ist.

Bei weiteren beispielhaften Ausführungsformen ist vorgesehen, dass das Gehäuse direkt an wenigstens einer Schicht des Bands befestigt ist bzw. zwischen verschiedenen Schichten des Bands angeordnet ist.

Bei weiteren beispielhaften Ausführungsformen ist vorgesehen, dass das Band eine Schnittstelle aufweist zum drahtlosen (z.B. induktiv) und/oder drahtgebundenen (z.B. mittels galvanischer Verbindung) Versorgen des Bandes und/oder wenigstens einer Komponente des Bandes mit Energie, beispielsweise zum Aufladen eines bzw. des Energiespeichers.

Weitere beispielhafte Ausführungsformen beziehen sich auf ein Verfahren zum Betreiben eines Bands zur Befestigung an einem Körper, beispielsweise an einer Extremität, beispielsweise eines Säugetiers und/oder einer Person, beispielsweise eines Armbands, wobei das Band eine Kommunikationseinrichtung aufweist, die dazu ausgebildet ist, eine kapazitive Datenübertragung, beispielsweise mit wenigstens einer weiteren Einrichtung, auszuführen, wobei das Verfahren aufweist: Ausführen einer kapazitiven Datenübertragung, beispielsweise mit der wenigstens einen weiteren Einrichtung.

Bei weiteren beispielhaften Ausführungsformen ist vorgesehen, dass Ausführen der kapazitiven Datenübertragung ein Senden von Daten mittels der kapazitiven Datenübertragung und/oder ein Empfangen von Daten mittels der kapazitiven Datenübertragung aufweist.

Bei weiteren beispielhaften Ausführungsformen kann ein Betrieb wenigstens einer Komponente des Bands beispielsweise basierend auf dem Ausführen der kapazitiven Datenübertragung, z.B. basierend auf dabei empfangenen Daten, gesteuert werden.

Weitere beispielhafte Ausführungsformen beziehen sich auf eine Verwendung des Bands gemäß den Ausführungsformen und/oder des Verfahrens gemäß den Ausführungsformen für wenigstens eines der folgenden Elemente: a) Empfangen von Daten von der wenigstens einen weiteren Einrichtung, b) Senden von Daten an die wenigstens eine weitere Einrichtung, c) Nutzen von Wärmeenergie des Körpers eines Trägers, beispielsweise für einen Betrieb wenigstens einer Komponente des Bands, d) Austauschen von Daten mit wenigstens einer Schließeinrichtung, e) Öffnen von Türen, f) Sammeln von Daten, beispielsweise biometrischen Daten, beispielsweise eines Trägers des Bands, g) Speichern und/oder Bereitstellen von Daten bezüglich einer Person, beispielsweise eines Patienten, beispielsweise von Daten betreffend eine Medikation des Patienten, h) Austauschen von Daten über eine Haut eines Trägers des Bands.

Weitere Merkmale, Anwendungsmöglichkeiten und Vorteile der Erfindung ergeben sich aus der nachfolgenden Beschreibung von Ausführungsbeispielen der Erfindung, die in den Figuren der Zeichnung dargestellt sind. Dabei bilden alle beschriebenen oder dargestellten Merkmale für sich oder in beliebiger Kombination den Gegenstand der Erfindung, unabhängig von ihrer Zusammenfassung in den Ansprüchen oder deren Rückbeziehung sowie unabhängig von ihrer Formulierung bzw. Darstellung in der Beschreibung bzw. in der Zeichnung.

In der Zeichnung zeigt:
- Fig. 1: schematisch ein vereinfachtes Blockdiagramm gemäß beispielhaften Ausführungsformen,
- Fig. 2: schematisch ein vereinfachtes Blockdiagramm gemäß beispielhaften Ausführungsformen,
- Fig. 3: schematisch eine Seitenansicht gemäß beispielhaften Ausführungsformen,
- Fig. 4A: schematisch ein vereinfachtes Flussdiagramm gemäß beispielhaften Ausführungsformen,
- Fig. 4B: schematisch ein vereinfachtes Flussdiagramm gemäß beispielhaften Ausführungsformen, und
- Fig. 5: schematisch Aspekte von Verwendungen gemäß weiteren beispielhaften Ausführungsformen.

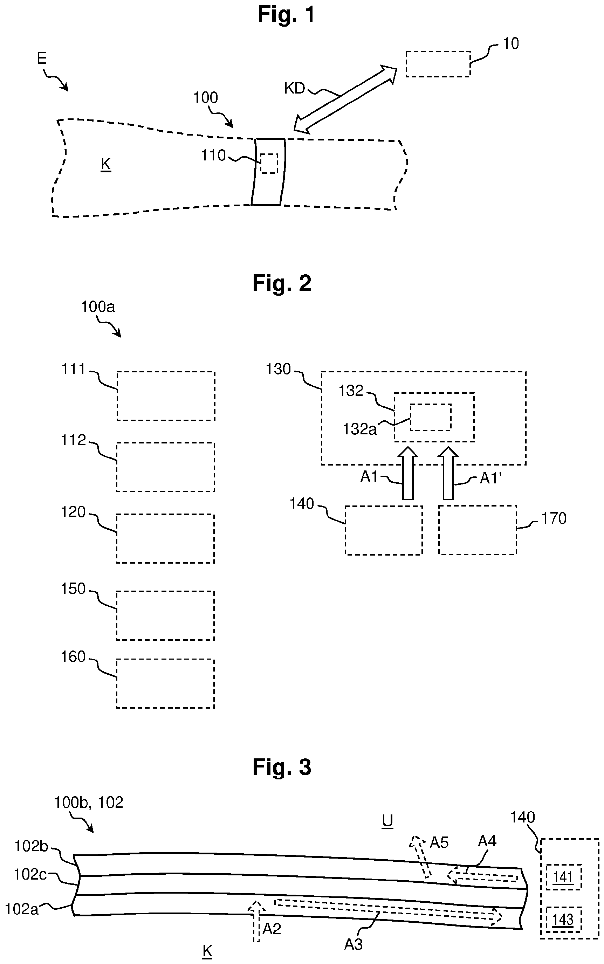

Beispielhafte Ausführungsformen, vgl. Fig. 1, beziehen sich auf ein Band 100 zur Befestigung an einem Körper K, beispielsweise an einer Extremität E, beispielsweise eines Säugetiers und/oder einer Person, beispielsweise Armband 100, aufweisend eine Kommunikationseinrichtung 110, die dazu ausgebildet ist, eine kapazitive Datenübertragung KD, beispielsweise mit wenigstens einer weiteren Einrichtung 10, auszuführen. Dadurch ist bei weiteren beispielhaften Ausführungsformen ein energieeffizienter Datenaustausch (z.B. Senden und/oder Empfangen von Daten) zwischen dem Band 100 und der wenigstens einer weiteren Einrichtung 10 ermöglicht.

Weitere beispielhafte Ausführungsformen stellen somit eine am Körper K tragbare bzw. mobile Einrichtung zur Ausführung einer kapazitiven Datenübertragung KD bereit.

Bei weiteren beispielhaften Ausführungsformen ist das Band 100 als Armband ausgebildet, also beispielsweise am bzw. um einen Arm E angeordnet tragbar.

Bei weiteren beispielhaften Ausführungsformen ist das Band 100 als Fußband ausgebildet, also beispielsweise am bzw. um einen Fuß bzw. ein Bein angeordnet tragbar.

Bei weiteren beispielhaften Ausführungsformen ist das Band 100 als Halsband ausgebildet, also beispielsweise am bzw. um den Hals angeordnet tragbar.

Bei weiteren beispielhaften Ausführungsformen kann das Band 100 z.B. von einer Person getragen werden.

Bei weiteren beispielhaften Ausführungsformen kann das Band z.B. von einem Tier, beispielsweise einem Säugetier, getragen werden.

Bei weiteren beispielhaften Ausführungsformen 100a, Fig. 2, ist vorgesehen, dass die Kommunikationseinrichtung 110 (Fig. 1) einen Sender 111 (Fig. 2) oder einen Transceiver 112 (Sendeempfänger) für die kapazitive Datenübertragung KD aufweist. Beispielsweise kann das Band 100 gemäß Fig. 1 die nachfolgend beispielhaft unter Bezugnahme auf Fig. 2 beschriebene Konfiguration aufweisen.

Bei manchen Ausführungsformen kann vorgesehen sein, dass das Band 100, 100a, beispielsweise ausschließlich, Daten mittels der kapazitiven Datenübertragung KD empfängt, z.B. von der Einrichtung 10 (Fig. 1).

Bei manchen Ausführungsformen kann vorgesehen sein, dass das Band 100, 100a, beispielsweise ausschließlich, Daten mittels der kapazitiven Datenübertragung KD sendet, z.B. an die Einrichtung 10 (Fig. 1).

Bei manchen Ausführungsformen kann vorgesehen sein, dass das Band 100, 100a zeitweise Daten mittels der kapazitiven Datenübertragung KD empfängt, z.B. von der Einrichtung 10 (Fig. 1), und dass das Band 100, 100a zeitweise Daten mittels der kapazitiven Datenübertragung KD sendet, z.B. an die Einrichtung 10.

Bei weiteren beispielhaften Ausführungsformen ist das Band 100, 100a dazu ausgebildet, Daten mit einer Mehrzahl (nicht gezeigt) von anderen Einrichtungen 10 auszutauschen.
Bei weiteren beispielhaften Ausführungsformen, Fig. 2, ist vorgesehen, dass das Band 100a wenigstens eine Antenne 120 für die kapazitive Datenübertragung KD (Fig. 1) aufweist.

Bei weiteren beispielhaften Ausführungsformen, Fig. 2, ist vorgesehen, dass das Band 100a eine Energieversorgungseinrichtung 130 zur zumindest zeitweisen Versorgung wenigstens einer Komponente 110, 111, 112, .. des Bands 100, 100a mit elektrischer Energie aufweist.

Bei weiteren beispielhaften Ausführungsformen ist vorgesehen, dass die Energieversorgungseinrichtung 130 einen Energiespeicher 132, beispielsweise zur Speicherung elektrischer Energie, aufweist, wobei beispielsweise der Energiespeicher 132 wenigstens einen Superkondensator 132a aufweist oder als Superkondensator ausgebildet ist.

Alternativ oder ergänzend zu dem wenigstens einen Superkondensator 132a kann bei weiteren beispielhaften Ausführungsformen auch wenigstens eine Batterie (nicht gezeigt) bzw. ein Akkumulator vorgesehen sein.

Bei weiteren beispielhaften Ausführungsformen ist vorgesehen, dass das Band 100, 100a einen thermoelektrischen Energiewandler 140 aufweist, der beispielsweise gemäß dem Seebeck-Effekt arbeitet, und/oder der beispielsweise dazu ausgebildet ist, einen bzw. den Energiespeicher 132 aufzuladen. Auf diese Weise kann z.B. die Körperwärme eines Trägers des Bandes 100, 100a genutzt werden, um elektrische Energie für den Betrieb der wenigstens einen Komponente 110, 111, 112, .. des Bands 100, 100a und/oder für das Aufladen des Energiespeichers 132 bereitzustellen.

Bei weiteren beispielhaften Ausführungsformen, vgl. die beispielhafte Konfiguration 100b gemäß Fig. 3, ist vorgesehen, dass das Band 100b einen Schichtaufbau 102 aufweist, wobei der Schichtaufbau 102 aufweist: eine erste Schicht 102a mit einer ersten Wärmeleitfähigkeit, eine zweite Schicht 102b mit einer zweiten Wärmeleitfähigkeit, und eine dritte Schicht 102c mit einer dritten Wärmeleitfähigkeit, wobei die dritte Schicht 102c zwischen der ersten Schicht 102a und der zweiten Schicht 102b angeordnet ist, wobei beispielsweise die erste Schicht 102a und die zweite Schicht 102b mit dem Energiewandler 140, beispielsweise thermisch leitfähig, verbindbar bzw. verbunden ist. Dies ermöglicht bei weiteren beispielhaften Ausführungsformen vorteilhaft eine effiziente Bereitstellung eines Temperaturunterschieds für den thermoelektrischen Energiewandler 140.

Bei weiteren beispielhaften Ausführungsformen ist vorgesehen, dass die erste Wärmeleitfähigkeit und/oder die zweite Wärmeleitfähigkeit größer ist als die dritte Wärmeleitfähigkeit, beispielsweise wenigstens um einen Faktor zehn, beispielsweise wenigstens um einen Faktor 100.

Bei weiteren beispielhaften Ausführungsformen ist vorgesehen, dass die erste Schicht 102a und die zweite Schicht 102b aus einem thermisch gut leitenden Material ausgebildet sind bzw. ein thermisch gut leitendes Material aufweisen. Bei weiteren beispielhaften Ausführungsformen können die erste und zweite Schicht 102a, 102b auch unterschiedliche Materialien aufweisen.

Bei weiteren beispielhaften Ausführungsformen ist vorgesehen, dass die dritte Schicht 102c ein thermisch nicht gut leitendes, also z.B. isolierendes, Material aufweist bzw. daraus ausgebildet ist.

Bei weiteren beispielhaften Ausführungsformen ist vorgesehen, dass die dritte Schicht 102c dazu ausgebildet ist die erste Schicht 102a thermisch von der zweiten Schicht 102b zu isolieren.

Bei weiteren beispielhaften Ausführungsformen ist vorgesehen, dass die erste Schicht 102a dazu ausgebildet ist, Wärmeenergie von einem Körper K eines Trägers des Bandes 100b zu dem Energiewandler 140, beispielsweise zu einem ersten Anschluss 141 des Energiewandlers hin, zu leiten, beispielsweise um den ersten Anschluss 141 des Energiewandlers zu erwärmen. Ein beispielhafter Fluss von Wärmeenergie aus dem Körper K durch die erste Schicht 102a des Bands 100b gemäß weiteren Ausführungsformen ist in Fig. 3 durch die Blockpfeile A2, A3 symbolisiert.

Bei weiteren beispielhaften Ausführungsformen ist vorgesehen, dass die zweite Schicht 102b dazu ausgebildet ist, Wärmeenergie von dem Energiewandler 140, beispielsweise von einem zweiten Anschluss 142 des Energiewandlers 140, weg zu leiten, beispielsweise zu einer Umgebung U, beispielsweise um den zweiten Anschluss 142 des Energiewandlers 140 zu kühlen. Ein beispielhafter Fluss von Wärmeenergie von dem zweiten Anschluss 142 durch die zweite Schicht 102b des Bands 100b hindurch zur Umgebung U gemäß weiteren Ausführungsformen ist in Fig. 3 durch die Blockpfeile A4, A5 symbolisiert.

Bei weiteren beispielhaften Ausführungsformen ist vorgesehen, dass das Band 100, 100a, 100b wenigstens eine Steuereinrichtung 150 (Fig. 2) zur zumindest zeitweisen Steuerung eines Betriebs wenigstens einer Komponente 110, 111, 112, .. aufweist. Bei weiteren beispielhaften Ausführungsformen ist vorgesehen, dass die Steuereinrichtung 150 einen Mikrocontroller oder einen programmierbaren Logikbaustein oder dergleichen aufweist.

Bei weiteren beispielhaften Ausführungsformen ist vorgesehen, dass das Band 100, 100a, 100b ein Gehäuse 160 (Fig. 2) aufweist, wobei wenigstens eine der Komponenten 110, 111, 112, 120, 130, 132, 132a, 140, 150 des Bands in und/oder an dem Gehäuse 160 angeordnet ist.

Bei weiteren beispielhaften Ausführungsformen ist vorgesehen, dass das Gehäuse 160 (Fig. 2) direkt an wenigstens einer Schicht 102a, 102b (Fig. 3) des Bands befestigt ist bzw. zwischen verschiedenen Schichten des Bands angeordnet ist.

Bei weiteren beispielhaften Ausführungsformen ist vorgesehen, dass das Band 100, 100a, 100b eine Schnittstelle 170 (Fig. 2) aufweist zum drahtlosen (z.B. induktiv) und/oder drahtgebundenen (z.B. mittels galvanischer Verbindung) Versorgen des Bandes und/oder wenigstens einer Komponente des Bandes mit Energie, beispielsweise zum Aufladen eines bzw. des Energiespeichers.

Ein beispielhaftes Aufladen des Energiespeichers 132 mittels des thermoelektrischen Energiewandlers 140 gemäß weiteren beispielhaften Ausführungsformen ist in Fig. 2 durch den Blockpfeil A1 symbolisiert, und ein beispielhaftes Aufladen des Energiespeichers 132 mittels der Schnittstelle 170 gemäß weiteren beispielhaften Ausführungsformen ist in Fig. 2 durch den Blockpfeil A1' symbolisiert

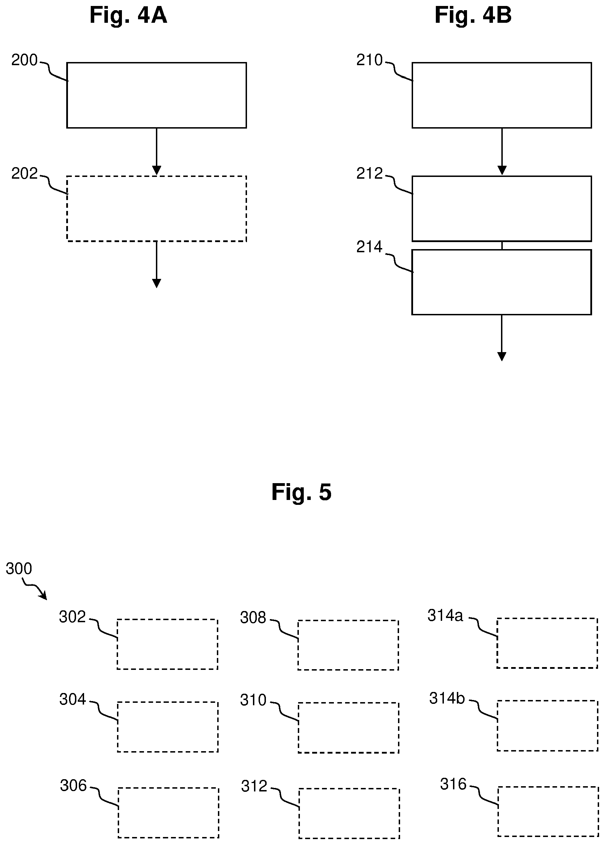

Weitere beispielhafte Ausführungsformen, Fig. 4A, beziehen sich auf ein Verfahren zum Betreiben eines Bands 100, 100a, 100b zur Befestigung an einem Körper K, beispielsweise an einer Extremität E, beispielsweise eines Säugetiers und/oder einer Person, beispielsweise eines Armbands, wobei das Band eine Kommunikationseinrichtung 110 aufweist, die dazu ausgebildet ist, eine kapazitive Datenübertragung KD (Fig. 1), beispielsweise mit wenigstens einer weiteren Einrichtung 10, auszuführen, wobei das Verfahren aufweist: Ausführen 200 (Fig. 4A) einer kapazitiven Datenübertragung, beispielsweise mit der wenigstens einen weiteren Einrichtung 10.

Bei weiteren beispielhaften Ausführungsformen ist vorgesehen, dass das Ausführen 200 der kapazitiven Datenübertragung KD ein Senden von Daten mittels der kapazitiven Datenübertragung KD und/oder ein Empfangen von Daten mittels der kapazitiven Datenübertragung KD aufweist.

Bei weiteren beispielhaften Ausführungsformen kann ein Betrieb wenigstens einer Komponente des Bands beispielsweise basierend auf dem Ausführen 200 der kapazitiven Datenübertragung, z.B. basierend auf dabei empfangenen Daten, gesteuert werden, vgl. den optionalen Block 202 gemäß Fig. 4A.

Fig. 4B zeigt ein vereinfachtes Flussdiagramm gemäß weiteren beispielhaften Ausführungsformen. Block 210 symbolisiert ein Empfangen von Daten mittels der kapazitiven Datenübertragung KD, Block 212 symbolisiert ein Verarbeiten der empfangenen Daten mittels der Steuereinrichtung 150, und Block 214 symbolisiert ein Senden von Daten mittels der kapazitiven Datenübertragung KD, z.B. basierend auf dem Verarbeiten 212. Ein Ablauf gemäß Fig. 4B ist bei weiteren beispielhaften Ausführungsformen beispielsweise dazu verwendbar, eine Kommunikation mit einer elektronischen Schließeinrichtung 10 (Fig. 1), z.B. für eine Tür, auszuführen, beispielsweise um eine Berechtigung zum Öffnen der Schließeinrichtung charakterisierende Daten an die elektronische Schließeinrichtung zu übermitteln.

Nachstehend sind weitere beispielhafte Aspekte und Ausführungsformen beschrieben, die jeweils einzeln für sich oder in Kombination miteinander mit wenigstens einer der vorstehend beispielhaft beschriebenen Ausführungsformen kombinierbar sind.

Bei weiteren beispielhaften Ausführungsformen ist das Band 100, 100a, 100b ein am Handgelenk einer Person tragbares Gerät und kann mittels der kapazitiven Datenübertragung KD z.B. zum Entriegeln von Türen verwendet werden. Dabei reicht es bei weiteren beispielhaften Ausführungsformen z.B. aus, einen Türgriff anzufassen bzw. die Hand in die Nähe des Türgriffs zu bewegen.

Bei weiteren beispielhaften Ausführungsformen kann das Band 100, 100a, 100b auch mit anderen Geräten und Einrichtungen als den beispielhaft genannten Türgriffen bzw. elektronischen Schlössern verwendet werden, z.B. mit Datenquellen und/oder Datensenken und/oder Gateways, die zur kapazitiven Datenübertragung ausgebildet sind.

Bei weiteren beispielhaften Ausführungsformen kann das Band 100, 100a, 100b z.B. in Seniorenheimen, Pflegeeinrichtungen, Krankenhäusern usw. verwendet werden.

Bei weiteren beispielhaften Ausführungsformen brauchen sich die Nutzer des Bands 100, 100a, 100b i.d.R. keine Gedanken mehr über z.B. das Öffnen von Türen machen. Beispielsweise lassen sich über ein Berühren bzw. in die Nähe bringen des Bandes und einer entsprechenden Einrichtung 10 Zutrittsdaten, wie sie z.B. für das Öffnen einer Tür verwendbar sind, übertragen, wodurch sich die Tür einfach und intuitiv öffnen lässt.

Bei weiteren beispielhaften Ausführungsformen verfügt das Band 100, 100a, 100b über eine Einrichtung 140 zum Umwandeln von Körperwärme in elektrische Energie. Damit wird z.B. die Betriebsdauer des Bands 100, 100a, 100b bzw. seiner (z.B. aktiven elektrischen bzw. elektronischen) Komponenten 110, 112, 112, 150 mitunter wesentlich erhöht bzw. das Band 100, 100a, 100b kann auch ohne Batterien bzw. Batteriewechsel auskommen.

Bei weiteren beispielhaften Ausführungsformen weist die Einrichtung 140 zum Umwandeln von Körperwärme in elektrische Energie bzw. ein entsprechender "Energie-Sammler" ("EnergyHarvester") z.B. wenigstens ein thermoelektrisches Element bzw. ein Peltier-Element bzw. Micro-Peltier-Element und z.B. das damit verbundene Band, z.B. den Schichtaufbau 102 gemäß Fig. 3, auf. Bei weiteren beispielhaften Ausführungsformen ist die erste Schicht 102a vergleichsweise stark wärmeleitend und kontaktiert z.B. die warme Oberfläche der Haut z.B. des Handgelenks direkt. Die eingesammelte Wärme-Energie verdichtet sich z.B. an der Kontaktstelle 141 zu dem Energiewandler, z.B. Micro-Peltier-Element 140. Auf der ersten Schicht 102a befindet sich bei weiteren beispielhaften Ausführungsformen eine wärmeisolierende Schicht 102c. Damit ein nutzbarer Wärmefluss zur Energieumwandlung (in elektrische Energie) entsteht, führt bei weiteren beispielhaften Ausführungsformen eine weitere vergleichsweise stark wärmeleitende Schicht 102b die verbliebene Energie an die Umgebung U ab. Die wärmeisolierende Schicht 102c verhindert z.B. einen thermischen Kurzschluss zwischen der Schicht 102a und der Schicht 102b.

Bei weiteren beispielhaften Ausführungsformen sind u.a. folgende weitere Anwendungen des Bands 100, 100a, 100b möglich: Einsammeln von Daten, die von z.B. am Körper getragenen Sensoren stammen (z.B. unter Verwendung der (z.B. menschlichen) Haut als Übertragungsmedium), Speichern von (z.B. Patienten-)Daten im Band bzw. in einem Speicher, auf den z.B. die Steuereinrichtung 150 (Fig. 2) zugreifen kann, der z.B. in die Steuereinrichtung 150 integriert ist, Speichern von Medikationsdaten, Abfrage der Daten durch ein Pflegepersonal, z.B. bei Berührung bzw. Annähern, Protokollieren von Handlungen, z.B. Pflegemaßnahmen, ...

Aufgrund der kapazitiven Datenübertragung KD, z.B. im Gegensatz zu einer funkbasierten Datenübertragung, ist das Band gemäß weiteren beispielhaften Ausführungsformen auch ohne Probleme in einem klinischen Umfeld bzw. im Bereich von Medizintechnik-Erzeugnissen bzw. -Systemen verwendbar. Weitere beispielhafte Ausführungsformen, Fig. 5, beziehen sich auf eine Verwendung 300 des Bands 100, 100a, 100b gemäß den Ausführungsformen und/oder des Verfahrens gemäß den Ausführungsformen für wenigstens eines der folgenden Elemente: a) Empfangen 302 von Daten von der wenigstens einen weiteren Einrichtung 10, b) Senden 304 von Daten an die wenigstens eine weitere Einrichtung 10, c) Nutzen 306 von Wärmeenergie des Körpers K eines Trägers, beispielsweise für einen Betrieb wenigstens einer Komponente des Bands, d) Austauschen 308 von Daten mit wenigstens einer Schließeinrichtung 10, e) Öffnen 310 von Türen, f) Sammeln 312 von Daten, beispielsweise biometrischen Daten, beispielsweise eines Trägers des Bands, g) Speichern 314a und/oder Bereitstellen 314b von Daten bezüglich einer Person, beispielsweise eines Patienten, beispielsweise von Daten betreffend eine Medikation des Patienten, h) Austauschen 316 von Daten über eine Haut eines Trägers des Bands.

## Patentansprüche

1. Band (100; 100a; 100b) zur Befestigung an einem Körper (K), beispielsweise an einer Extremität (E), beispielsweise eines Säugetiers und/oder einer Person, beispielsweise Armband, aufweisend eine Kommunikationseinrichtung (110), die dazu ausgebildet ist, eine kapazitive Datenübertragung (KD), beispielsweise mit wenigstens einer weiteren Einrichtung (10), auszuführen.

2. Band (100) nach Anspruch 1, wobei die Kommunikationseinrichtung (110) einen Sender (111) oder einen Transceiver (112) für die kapazitive Datenübertragung (KD) aufweist.

3. Band (100) nach wenigstens einem der vorstehenden Ansprüche, aufweisend wenigstens eine Antenne (120) für die kapazitive Datenübertragung (KD).

4. Band (100) nach wenigstens einem der vorstehenden Ansprüche, aufweisend eine Energieversorgungseinrichtung (130) zur zumindest zeitweisen Versorgung wenigstens einer Komponente (110, 120, 130, 150) des Bands (100) mit elektrischer Energie.

5. Band (100) nach Anspruch 4, wobei die Energieversorgungseinrichtung (130) einen Energiespeicher (132), beispielsweise zur Speicherung elektrischer Energie, aufweist, wobei beispielsweise der Energiespeicher (132) wenigstens einen Superkondensator (132a) aufweist oder als Superkondensator (132a) ausgebildet ist.

6. Band (100) nach wenigstens einem der vorstehenden Ansprüche, aufweisend einen thermoelektrischen Energiewandler (140), der beispielsweise gemäß dem Seebeck-Effekt arbeitet, und/oder der beispielsweise dazu ausgebildet ist, einen bzw. den Energiespeicher (132) aufzuladen.

7. Band (100) nach Anspruch 6, wobei das Band (100) einen Schichtaufbau (102) aufweist, wobei der Schichtaufbau (102) aufweist: eine erste Schicht (102a) mit einer ersten Wärmeleitfähigkeit, eine zweite Schicht (102b) mit einer zweiten Wärmeleitfähigkeit, und eine dritte Schicht (102c) mit einer dritten Wärmeleitfähigkeit, wobei die dritte Schicht (102c) zwischen der ersten Schicht (102a) und der zweiten Schicht (102b) angeordnet ist, wobei beispielsweise die erste Schicht (102a) und die zweite Schicht (102b) mit dem Energiewandler (140), beispielsweise thermisch leitfähig, verbindbar bzw. verbunden ist.

8. Band (100) nach Anspruch 7, wobei die erste Wärmeleitfähigkeit und/oder die zweite Wärmeleitfähigkeit größer ist als die dritte Wärmeleitfähigkeit, beispielsweise wenigstens um einen Faktor zehn, beispielsweise wenigstens um einen Faktor 100.

9. Band (100) nach wenigstens einem der Ansprüche 7 bis 8, wobei die dritte Schicht (102c) dazu ausgebildet ist die erste Schicht (102a) thermisch von der zweiten Schicht (102c) zu isolieren.

10. Band (100) nach wenigstens einem der Ansprüche 7 bis 9, wobei die erste Schicht (102a) dazu ausgebildet ist, Wärmeenergie von einem Körper (K) eines Trägers des Bandes (100) zu dem Energiewandler (140), beispielsweise zu einem ersten Anschluss (141) des Energiewandlers (140) hin, zu leiten, und/oder wobei die zweite Schicht (102b) dazu ausgebildet ist, Wärmeenergie von dem Energiewandler (140), beispielsweise von einem zweiten Anschluss (142) des Energiewandlers (140), weg zu leiten, beispielsweise zu einer Umgebung (U).

11. Band (100) nach wenigstens einem der vorstehenden Ansprüche, aufweisend wenigstens eine Steuereinrichtung (150) zur zumindest zeitweisen Steuerung eines Betriebs wenigstens einer Komponente (110, 120, 130, 140).

12. Band (100) nach wenigstens einem der vorstehenden Ansprüche, wobei das Band (100) ein Gehäuse (160) aufweist, wobei wenigstens eine der Komponenten (110, 120, 130, 140, 150) des Bands (100) in und/oder an dem Gehäuse (160) angeordnet ist.

13. Band (100) nach wenigstens einem der vorstehenden Ansprüche, aufweisend eine Schnittstelle (170) zum drahtlosen und/oder drahtgebundenen Versorgen des Bandes (100) und/oder wenigstens einer Komponente des Bandes (100) mit Energie, beispielsweise zum Aufladen eines bzw. des Energiespeichers (132).

14. Verfahren zum Betreiben eines Bands (100; 100a; 100b) zur Befestigung an einem Körper (K), beispielsweise an einer Extremität (E), beispielsweise eines Säugetiers und/oder einer Person, beispielsweise eines Armbands, wobei das Band (100) eine Kommunikationseinrichtung (110) aufweist, die dazu ausgebildet ist, eine kapazitive Datenübertragung (KD), beispielsweise mit wenigstens einer weiteren Einrichtung (10), auszuführen, wobei das Verfahren aufweist: Ausführen (200) einer kapazitiven Datenübertragung (KD), beispielsweise mit der wenigstens einen weiteren Einrichtung (10).

15. Verwendung (300) des Bands (100; 100a; 100b) nach wenigstens einem der Ansprüche 1 bis 13 und/oder des Verfahrens nach Anspruch 14 für wenigstens eines der folgenden Elemente: a) Empfangen (302) von Daten von der wenigstens einen weiteren Einrichtung (10), b) Senden (304) von Daten an die wenigstens eine weitere Einrichtung (10), c) Nutzen (306) von Wärmeenergie des Körpers (K) eines Trägers, beispielsweise für einen Betrieb wenigstens einer Komponente des Bands, d) Austauschen (308) von Daten mit wenigstens einer Schließeinrichtung, e) Öffnen (310) von Türen, f) Sammeln (312) von Daten, beispielsweise biometrischen Daten, beispielsweise eines Trägers des Bands, g) Speichern (314a) und/oder Bereitstellen (314b) von Daten bezüglich einer Person, beispielsweise eines Patienten, beispielsweise von Daten betreffend eine Medikation des Patienten, h) Austauschen (316) von Daten über eine Haut eines Trägers des Bands.
